# EUROPEAN PATENT SPECIFICATION

(11) **EP 1 948 291 B1**
(45) Date of publication and mention of the grant of the patent: **14.08.2013**
(21) Application number: 06846238.1
(22) Date of filing: 07.11.2006
(51) Int. Cl.: A61F 5/08, A61F 5/56, A61F 13/02, A61M 29/00

(54) **DELAYED ACTION SPRING FORCE ELEMENT FOR NASAL DILATORS**
FEDERKRAFTELEMENT MIT VERZÖGERTER BETÄTIGUNG FÜR NASALE DILATOREN
ELEMENT A EFFET DE RESSORT A ACTION RETARDEE POUR DILATATEURS NASAUX

(30) Priority: 07.11.2005 US 734676 P
(43) Date of publication of application: 30.07.2008
(73) Proprietor: CNS, INC., Minneapolis, MN 55439-0802 (US)
(72) Inventor: FENTON, Gustav, R., Minneapolis, MN 55409 (US)
(74) Representative: Walker, Ralph Francis
(86) International application number: PCT/US2006/060587
(87) International publication number: WO 2007/056715

(56) References cited:
- EP-A1- 1 033 118
- WO-A1-98/15242
- WO-A2-2005/020845
- US-A- 5 476 091
- US-A- 5 653 224
- US-A1- 2002 000 227
- US-A1- 2003 000 521
- US-B1- 6 470 883

## Description

### INTRODUCTION

External nasal dilators, useful for alleviating snoring and other breathing-delated disorders, are called upon to perform two opposing functions. First, the dilator needs to attach securely to the skin of the nose. Second, the dilator applies outward force to open the nasal valve region. If the initial attachment force is insufficient then the second outward force will cause the dilator to detach. In current nasal dilators, adhesives, such as acrylate adhesives, set up for adherence relatively quickly such that the outward spring force does not typically cause detachment.

US 5,653,224 discloses a nasal dilator with areas of different adhesive force at different locations. WO-A-98/15242 discloses a nasal dilator with a connecting member and tensile member kept separate so they can be moved relative to each other. WO-A-2005/020845 discloses a nasal dilator that uses multiple filaments to provide a resilient region. US 6,470,883 discloses a nasal dilator with a laminated resilient member. US-A-2002/0000227 discloses a tissue dilator device which uses a "memory metal" as a resilient member.

### BACKGROUND

There are, however, problems with the use of a pressure sensitive acrylate adhesive. For instance, the acrylate adhesive's strong adherence during removal (when less adherence would be kinder to the skin), its loss of adhesiveness during sweating, and its potential to block pores or cause irritation are all problems encountered when using fast-setting adhesives. While these issues are minor, they are widely acknowledged, since acrylate pressure sensitive adhesives are used extensively in commercially available wound care bandages.

For pressure sensitive adhesive applications, hydrocolloid adhesives are an alternative to acrylates. Initially, however, the hydrocolloids do not provide enough adhesiveness. As used herein, the term "adhesiveness" will be defined as the grams of force needed to remove the strip from the skin. The following is a comparison of typical adhesiveness over time:
- Acrylate adhesiveness on human skin:
   o ∼45 grams per inch initially,
   o ∼100 grams per inch at four hours and
   o - 200 grams per inch at 24 hours.
- Hydrocolloid adhesiveness on human skin:
   o - 25 grams per inch initially,
   o ∼100 grams per inch at 20 to 30 minutes and
   o ∼140 grams per inch at 24 hours.

The acrylate adhesiveness (∼ 45 grams per inch) initially is adequate to counteract the external nasal dilator spring force, but the hydrocolloid's initial adhesiveness (∼ 25 grams per inch initially) is not sufficient to counteract the spring force. Between 5 and 20 minutes, the hydrocolloid's adhesiveness increases to a point where it can counteract the spring force.

Hydrocolloid materials offer good adhesion, ease of removal and gentleness to the skin. As noted above, the issue with a hydrocolloid adhesive is that its initial adhesiveness is insufficient to counteract the external nasal dilator's spring force, which can lead to detachment of the nasal dilator from the skin. The initial low adhesion builds to a significant amount as the hydrocolloid material warms up and adapts to the skin's surface, after which the adhesion is appropriate for countering the typical nasal dilator spring forced This period of growing adhesion may be anywhere from five minutes to an hour.

Therefore, there is a need for a nasal dilator adhesion system in which one could provide enough time for the hydrocolloid adhesive to establish adequate adhesion by delaying the outward detaching/dilating force that opens the nasal valve, i.e., the nasal dilator spring force.

The present invention provides a nasal dilator according to claim 1.

### SUMMARY OF THE INVENTION

The present nasal dilator has a spring force element and an adhesive element The spring force element has a latent state of minimal spring force, and is configured to be activated to cause the spring force to increase to an amount sufficient to dilate nasal tissues.

### DESCRIPTION OF THE INVENTION

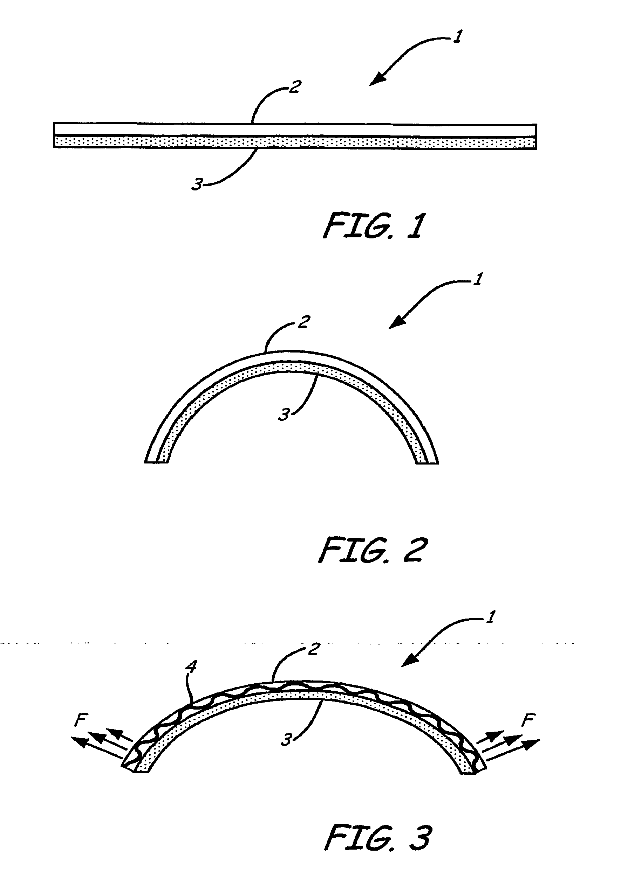

The present invention comprises two elements to form a nasal dilator strip 1, as shown in Fig. 1. The spring force layer 2 has minimal spring force in its latent, typically flattened state as shown in Fig. 1. The skin contact layer 3 provides a base for the adhesion to the nose and is a hydrocolloid. An optional fabric or mesh framework 4, as shown in Fig. 3, can be incorporated into the spring force layer 2 to provide a lattice that can be both contracted and stiffened.

Although the embodiments contained herein describe these elements in the form of layers, other constructions, such as embedded elements, are within the scope of the present invention.

Nasal dilator strip 1 is attached to the nose by bending strip 1 to conform to the outer surface of the nose, as shown in Fig. 2. Upon some sort of activation process, the spring force layer 2 contracts, thereby gaining an outward spring force F sufficient to act as a nasal dilator, which is shown in Fig. 3. This activation would take place over a period preferably of about five to about thirty minutes after placing the nasal dilator strip 1 on the user's nose. This time period allows the skin contact layer 3 time to fully establish its adhesion to the nose skin prior to the outward force F reaching its maximum. Materials in the spring force layer 2 have the property of contracting upon exposure to air, light, moisture, heat, or a stress or pressure force, or any combination thereof.

The spring force layer 2 preferably has the following properties:
■ Intimately connected to the skin contact layer 3,
■ Minimal inherent lateral force when in its latent storage state and when ftrst attached to the nose,
■ An activation mechanism or process whereby adequate lateral force (to open the nasal valve or otherwise dilate nasal tissues) can be developed, typically by contraction, within a period of about five to about thirty minutes after attachment to the nose,
■ Made of medical grade materials that are non-irritating to the skin,
■ Operates in an automatic manner such that the user does not need to intervene or intervenes minimally after starting the process by attaching the strip to his or her nose.

Several methods and embodiments are described below whereby the spring force layer 2 can be constructed to achieve the properties noted above.
■ Liner protection: The spring force layer 2 can be made of a liquid, gel or other material that is covered with a protective liner. To use the nasal dilator strip 1, the liner is removed, which exposes the spring force layer 2 to air or light. The exposure to air or light causes the material to dry or otherwise change and contract, which exerts outward force on the nose for dilation. An optional fabric or mesh framework 4, as shown in Fig. 3, can be incorporated into the spring force layer 2 to provide a lattice that can be both contracted and stiffened.
■ Encapsulation: The spring force layer 2 can be made of encapsulated liquid (for
   example, an adhesive) that could be activated by crushing the capsules and exposing the liquid. This embodiment is similar to "scratch and sniff" fragrance delivery systems. Once exposed, the encapsulated material contracts, causing the nose to dilate. An optional fabric or mesh framework 4, as shown in Fig. 3, can be incorporated into the spring force layer 2 to provide a lattice that can be both contracted and stiffened.
■ Bending stress activation: The spring force layer 2 can be made of material that is inactivate in its flattened storage state. The bending/stressing of the strip across the nose causes it to become activated to begin its contraction process. An optional fabric or mesh framework 4, as shown in Fig. 3, can be incorporated into the spring force layer 2 to provide a lattice that can be both contracted and stiffened.
■ Water activation: The spring force layer 2 can be made of material that is pliable under dry conditions, but contracts when it absorbs water. The water may be introduced by the user (for example, by wetting the outside of the strip after it is attached to the nose). Alternatively, a hydrocolloid material which absorbs water quite well and could act as a reservoir for the water to activate the spring force layer 2.
■ Heat activation: The spring force layer 2 can be made of material that is pliable under cool, storage conditions, but contracts when it is exposed to air and heated to body temperature. This embodiment may require a protective liner to be removed from the top of the spring force layer 2 to prevent activation during hot storage conditions. Alternatively, the strip can be sealed from access to air during storage.

Although the foregoing fully describes and discloses the nasal dilator strip of the present invention, it is not intended to limit the scope of the present invention, which is defined by the following claims.

## Claims

1. A nasal dilator (1) for application to a nose to dilate nasal tissues, comprising a spring force layer (2) comprising a hydrocolloid adhesive, and a skin contact layer *(3),* ***characterised* in that** the spring force layer (2) includes a material having an initial latent state of minimal spring force and that subsequent to application to nasal tissues and after sufficient time for the hydrocolloid adhesive to establish adequate adhesion to the nasal tissues is configured to be activated to cause the spring force to increase to an active state of greater spring force, wherein either:
the spring force layer (2) is made of a liquid, gel or other material that is covered with a removable protective liner which when removed exposes the spring force layer to air or light, and this exposure to air or light causes the material to dry or otherwise change and contract to exert outward force on the nose for dilation, or;
the spring force layer (2) is made of encapsulated liquid that is activated by crushing the capsules and exposing the liquid so that once exposed the encapsulated material contracts, causing the nose to dilate, or;
the spring force layer (2) is made of material that is inactive in its flattened storage state, and the bending/stressing of the material across the nose causes the material to become activated to begin a contraction process, or;
the spring force layer (2) is made of a material that is pliable under cool storage conditions but contracts when it is exposed to air and heated to body temperatures.

2. The nasal dilator (1) of claim 1 ***characterised* in that** the active state is achieved about 5 to about 30 minutes after the nasal dilator (1) is applied to the nose.

3. The nasal dilator (1) of claim 1 ***characterised* in that** the greater spring force is sufficient to open a nasal valve and dilate tissue.

4. The nasal dilator (1) of claim 1 ***characterised* in that** the hydrocolloid adhesive has an initial low adhesiveness and a subsequent greater adhesiveness.

5. The nasal dilator (1) of claim 4 ***characterised* in that** the initial adhesiveness is sufficient to cause the nasal dilator (1) to adhese to the nose.

6. The nasal dilator (1) of claim 4 ***characterised* in that** the subsequent greater adhesiveness is sufficient for the nasal dilator (1) to remain adhered to the nose upon dilation of the nasal tissues by the spring force layer (2).

7. The nasal dilator (1) of claim 1 ***characterised* in that** the spring force layer (2) further comprises a lattice (4) capable of being contracted and stiffened.

## Patentansprüche

1. Nasaldilatator (1) zum Anwenden auf eine Nase, um Nasalgewebe aufzuweiten, mit einer Federkraftlage (2), die ein Hydrokolloidhaftmittel aufweist, und einer Hautkontaktlage (3), **dadurch gekennzeichnet, dass** die Federkraftlage (2) ein Material mit einem initial ruhenden Zustand minimaler Federkraft aufweist, das nach dem Aufbringen auf das Nasalgewebe und nach ausreichender Zeit für das Hydrokolloidhaftmittel eine adäquate Haftung an dem Nasalgewebe herstellt, eingerichtet ist, aktiviert zu werden, um zu verursachen, dass sich die Federkraft auf einen aktiven Zustand größerer Federkraft erhöht, wobei entweder
die Federkraftlage (2) aus einer Flüssigkeit, einem Gel oder einem anderen Material hergestellt ist, das mit einem entfernbaren schützenden Einsatz bedeckt ist, der nach Entfernung die Federkraftlage Luft oder Licht aussetzt und dieses Aussetzen an Luft oder Licht verursacht, dass das Material trocknet oder sich anderweitig ändert und sich zusammenzieht, um eine nach außen gerichtete Kraft auf die Nase zur Aufweitung auszuüben, oder
die Federkraftlage (2) aus eingekapselter Flüssigkeit hergestellt ist, die durch Zerdrücken der Kapseln und Aussetzen der Flüssigkeit aktiviert wird, sodass das eingekapselte Material einmal ausgesetzt kontrahiert und verursacht, dass sich die Nase aufweitet, oder
die Federkraftlage (2) aus einem Material hergestellt ist, das in seinem flachen Aufbewahrungszustand inaktiv ist und das Biegen/Spannen des Materials über der Nase verursacht, dass das Material aktiviert wird, um einen Kontrahierungsprozess zu beginnen, oder
die Federkraftlage (2) aus einem Material hergestellt ist, das unter kalten Aufbewahrungsbedingungen biegsam ist, sich jedoch zusammenzieht, wenn es Luft ausgesetzt und auf Körpertemperatur aufgewärmt wird.

2. Nasaldilatator (1) nach Anspruch 1, **dadurch gekennzeichnet, dass** der aktive Zustand in etwa 5 bis in etwa 30 min nachdem der Nasaldilatator (1) auf der Nase aufgebracht ist, erreicht wird.

3. Nasaldilatator (1) nach Anspruch 1, **dadurch gekennzeichnet, dass** die höhere Federkraft ausreicht, um eine Nasalklappe zu öffnen und Gewebe aufzuweiten.

4. Nasaldilatator (1) nach Anspruch 1, **dadurch gekennzeichnet, dass** das Hydrokolloidhaftmittel initial eine niedrige Haftung und nachfolgend eine höhere Haftung aufweist.

5. Nasaldilatator (1) nach Anspruch 4, **dadurch gekennzeichnet, dass** die initiale Haftfähigkeit ausreichend ist, um zu verursachen, dass der Nasaldilatator (1) an der Nase haftet.

6. Nasaldilatator (1) nach Anspruch 4, **dadurch gekennzeichnet, dass** die nachfolgende größere Haftfähigkeit für den Nasaldilatator (1) ausreichend ist, um bei Aufweitung des Nasalgewebes durch die Federkraftlage (2) an der Nase haften zu bleiben.

7. Nasaldilatator (1) nach Anspruch 1, **dadurch gekennzeichnet, dass** die Federkraftlage (2) des Weiteren ein Gitter (4) aufweist, das imstande ist, sich zusammenzuziehen und versteift zu werden.

## Revendications

1. Dilatateur nasal (1) pour application sur un nez pour dilater les tissus nasaux, comprenant une couche à effet de ressort (2) comprenant un adhésif hydrocolloïdal, et une couche de contact cutané (3), **caractérisé en ce que** la couche à effet de ressort (2) comprend un matériau ayant un état latent initial d'effet de ressort minimal et qui, après application sur les tissus nasaux et après une durée suffisante pour que l'adhésif hydrocolloïdal établisse une adhérence adéquate sur les tissus nasaux, est configuré pour être activé de façon à entraîner l'augmentation de l'effet de ressort à un état actif d'effet de ressort supérieur, dans lequel soit :
la couche à effet de ressort (2) est constituée d'un liquide, d'un gel ou d'un autre matériau qui est recouvert avec un revêtement protecteur amovible qui, lorsqu'il est retiré, expose la couche à effet de ressort à l'air ou à la lumière, et cette exposition à l'air ou à la lumière entraîne un séchage ou un changement d'une autre façon et une contraction du matériau de façon à exercer une force vers l'extérieur sur le nez pour sa dilatation, soit ;
la couche à effet de ressort (2) est constituée d'un liquide encapsulé qui est activé par écrasement des capsules et exposition du liquide de sorte que, une fois exposé, le matériau encapsulé se contracte, entraînant la dilatation du nez, soit ;
la couche à effet de ressort (2) est constituée d'un matériau qui est inactif dans son état de stockage aplati et la courbure/la contrainte du matériau sur le nez entraîne l'activation du matériau de façon à commencer un processus de contraction, soit ;
la couche à effet de ressort (2) est constituée d'un matériau qui est souple dans des conditions de stockage froides mais se contracte lorsqu'il est exposé à l'air et est chauffé aux températures corporelles.

2. Dilatateur nasal (1) selon la revendication 1, **caractérisé en ce que** l'état actif est obtenu environ 5 à environ 30 minutes après que le dilatateur nasal (1) est appliqué sur le nez.

3. Dilatateur nasal (1) selon la revendication 1, **caractérisé en ce que** l'effet de ressort supérieur est suffisant pour ouvrir une valve nasale et dilater les tissus.

4. Dilatateur nasal (1) selon la revendication 1, **caractérisé en ce que** l'adhésif hydrocolloïdal a une adhésivité initiale faible et une adhésivité ultérieure supérieure.

5. Dilatateur nasal (1) selon la revendication 4, **caractérisé en ce que** l'adhésivité initiale est suffisante pour entraîner l'adhérence du dilatateur nasal (1) sur le nez.

6. Dilatateur nasal (1) selon la revendication 4, **caractérisé en ce que** l'adhésivité ultérieure supérieure est suffisante pour que le dilatateur nasal (1) reste collé sur le nez après dilatation des tissus nasaux par la couche à effet de ressort (2).

7. Dilatateur nasal (1) selon la revendication 1, **caractérisé en ce que** la couche à effet de ressort (2) comprend en outre un treillis (4) pouvant être contracté et rigidifié.
